# EUROPEAN PATENT SPECIFICATION

(11) **EP 2 043 506 B1**
(45) Date of publication and mention of the grant of the patent: **09.07.2014**
(21) Application number: 07784667.3
(22) Date of filing: 23.07.2007
(51) Int. Cl.: A61B 5/00, G01D 5/353, G01L 1/24, G01L 11/02, G02B 27/44

(54) **AN APPARATUS FOR PRESSURE SENSING**
VORRICHTUNG ZUR DRUCKMESSUNG
APPAREIL DE DÉTECTION DE PRESSION

(30) Priority: 26.07.2006 AU 2006904021
(43) Date of publication of application: 08.04.2009
(73) Proprietor: Commonwealth Scientific and Industrial Research Organisation, Campbell, Australian Capital Territory 2612 (AU)
(72) Inventor: ARKWRIGHT, John William, Campbell, Australian Capital Territory 2612 (AU); DOE, Simon Nicholas, Campbell, Australian Capital Territory 2612 (AU); JARVIS, Brian Laurence, Campbell, Australian Capital Territory 2612 (AU); HARTMANN, Andreas, Beaconsfield, Western Australia 6162 (AU)
(74) Representative: Watterson, Peer Marten John
(86) International application number: PCT/AU2007/001018
(87) International publication number: WO 2008/011663

(56) References cited:
- WO-A1-2006/094351
- WO-A1-2006/094352
- WO-A1-2006/094353
- GB-A- 2 371 359
- GB-A- 2 371 360
- US-A1- 2001 021 843
- US-A1- 2004 114 849
- US-B1- 6 218 661
- US-B1- 6 233 374

## Description

### Field of the Invention

The present invention broadly relates to an apparatus for pressure sensing.

### Background of the Invention

The human body has many regions in which pressure differences cause matter to move. For example, the human heart pumps blood through the body. Muscles around the alimentary canal apply a pressure to the channel which moves food from the mouth into the stomach. Further, a pressure increase in a portion of the body may be caused by a chemical reaction such as the development of a gas in an enclosed body cavity.

Monitoring pressures in the human body can provide important information about the function of the human body and can be used to detect disorders and diseases or can be used to control a recovery from a disease.

For example, dysphagia, which is a disorder that causes difficulty in swallowing, typically affects infants and elderly people and is especially prevalent in post-stroke patients. It is difficult to diagnose this disease and diagnostic tools are often very uncomfortable for the patient.

A multi-bore catheter tube is commonly used for diagnosis of this disorder and the multi-bore catheter is inserted into the oesophagus. The exit ports of the bores of the catheter are positioned at different locations along the catheter and a steady flow of water exits through each port. Measurement of the hydraulic water pressure at an input of each bore gives an indication of the pressure distribution in the oesophagus and therefore can be used to diagnose the disorder.

Another method of in-vivo pressure measurement involves usage of a series of piezoelectric or electro- mechanical devices. Such devices typically are expensive and require a relatively large number of electrical wires to be contained in a catheter which consequently is of relatively large thickness. The device is inserted through the nose of the patient and its relatively large diameter results in discomfort for the patient.

US 2 001 021 843 discloses a catheter using an optical fibre with a number of Bragg gratings at joints of the catheter.

The present invention provides an improved technological solution.

### Summary of the Invention

The present invention provides in a first aspect an apparatus for distributed pressure sensing in accordance with appended claim 1,

The apparatus may comprise at least one rigid member that is attached at attachment regions between which a sensing region of at least one Bragg grating is defined so that a series of the sensing regions is formed, wherein the at least one rigid member is arranged so that a strain in the sensing region is not directly influenced by a change in strain of the light guide outside the sensing region.

As pressure differences between adjacent internal spaces are reduced, a local increase in internal pressure caused by a suitable movement of movable wall portion is reduced, which increases the movability of the movable wall portion and thereby the sensitivity of the apparatus.

The change in strain of each Bragg grating causes a change in an optical response of the Bragg grating to light that is in use guided to each Bragg grating so that the changes in external pressure at the location of the Bragg gratings can be detected. Because the apparatus typically is arranged so that a strain of any one of the sensing regions is not directly or significantly influenced by a change in strain of the light guide outside that sensing region, it is possible to measure the pressure at the position of a plurality of sensing region largely independent from each other and thereby measure a pressure distribution. This also allows to measure pressure at a plurality of sensing regions independently of each other in an environment that inherently applies an axial strain to the measuring device, such as the human oesophagus.

For example, the sensing regions with the at least one rigid member may be positioned in a flexible tube and the internal space at each sensing region may be portions of the internal space of the flexible tube. The internal spaces at adjacent sensing regions typically are not separated by an interior wall portion.

The moveable wall portions may be wall portions of the tube which may be a tubular diaphragm. The tube typically is formed from a polymeric material such as silicone and may be positioned in a catheter which comprises an outer biocompatible surface or which is formed from a biocompatible flexible material.

Alternatively, the tube may also itself be provided in form of such a catheter and the moveable wall portions may be wall portions of the catheter.

The tube may have open end-portions or at least one end-portion of the tube may be closed. The apparatus typically is arranged for in-vivo pressure measurements and the tube typically has one open end that is arranged to be located outside a body during the in-vivo pressure measurements so that the internal spaces at the Bragg gratings are in fluidal communication with a space outside the body. The other end of the tube typically is closed. If the tube would have two closed ends, temperature fluctuations may cause pressure variations within the tube and such pressure variations could affect the strain of the at least one Bragg grating and thereby the accuracy of the in-vivo pressure measurements. The open end of the tube substantially avoids such pressure variations within the tube.

Each moveable wall portion may also be an individual diaphragm and the individual diaphragms with the at least one rigid member together with at least a portion of the light guide may be positioned in a catheter for insertion into a body lumen.

The at least one rigid member typically is attached to the light guide at attachment regions at either end of each sensing region.

The same rigid member may be attached to the light guide at attachment regions associated with more than one Bragg gratings. In one specific embodiment, the apparatus comprises one rigid member that is attached at all attachment regions. For example, the rigid member may be a rod that is bent in a suitable manner and/or that comprises arms that are attached to the attachment regions in a manner so that the light guide can move in response to a change in pressure at one of the sides of the moveable wall portions relative to a pressure at the other side of the moveable wall portions.

Alternatively, each sensing region may be associated with an individual rigid member. In this case each rigid member may be of any suitable shape, but typically are of a substantially V-, C- or U- cross-sectional shape.

Further, the apparatus may comprise a portion comprising an X-ray opaque material which enables imaging of the position of the apparatus in the human body. For example, the apparatus may be arranged for positioning in the oesophagus of the human body for distributed pressure measurement in the oesophagus. With the above-described apparatus it is typically possible to measure a distributed pressure in the oesophagus in a manner such that a swallowing action or food travelling past a selected region does not significantly influence a pressure reading at another sensing region by an axial strain originating from the selected sensing region.

Each Bragg grating of the series typically is arranged to give a different optical response so that light reflected from each Bragg grating is wavelength division multiplexed. As each Bragg grating gives a different response, it is possible to associate a particular pressure change with a respective position.

In a variation of this embodiment at least some of the Bragg gratings are substantially identical and give the same response if the strain conditions are the same. Using time domain reflectometry techniques, the position of a particular Bragg grating may be estimated from a time at which an optical response is received.

The light guide may comprise one optical fibre which may comprise portions that are spliced together. The optical light guide typically is attached at the attachment regions, but typically is flexible at regions between adjacent rigid members so that the apparatus is articulated.

The force on each Bragg grating typically is a force on a side portion of each Bragg grating. The apparatus typically is arranged so that the forces on the side portions are applied from one side of each Bragg grating at the sensing region. The apparatus may be arranged so that the forces are applied in any transversal or non-axial direction of the Bragg grating, but the apparatus typically is arranged so that the forces are applied in a direction that is substantially perpendicular to an axis of respective Bragg gratings.

The apparatus may have a normal operating temperature and pressure range at which the Bragg gratings are distorted into respective internal spaces or alternatively may be distorted in an opposite direction. The apparatus may be arranged so that a temperature related change in a property of the moveable wall portions, which typically are positioned adjacent respective Bragg gratings, reduces the temperature related change in the optical response of the Bragg gratings. In this case the apparatus has the particular advantage that the moveable wall portion has a dual function, namely reducing the temperature related change in the optical period of the Bragg grating and causing a force on the Bragg grating in response to an external pressure change.

The apparatus may be used for pressure measurements in any environment, including for example in-vivo-environments, laboratories and wind tunnels.

The Bragg gratings typically are positioned on respective moveable wall portions. Alternatively, the Bragg gratings may be positioned within the moveable wall portions.

The apparatus may be arranged so that the optical response from each Bragg grating can be detected by detecting light that is reflected back from the Bragg gratings. In this case the light guide typically is arranged so that the light is guided to and from the Bragg gratings by the same optical fibre portion.

The apparatus may also be arranged so that the optical responses from the Bragg gratings can be detected by detecting light that is transmitted through the Bragg gratings. In this case the light guide typically comprises at least one optical fibre for guiding the light to the Bragg gratings and at least one other optical fibre for guiding the light from the Bragg gratings.

The light guide may comprise an optical fibre such as a single mode optical fibre in which the Bragg gratings may have been written. As optical fibres are known to cause very little signal loss per length, the apparatus can have a relatively long optical fibre lead and an optical analyser for analysing the response from the at least one Bragg grating may be remote from the at least one Bragg grating, such as 1m, 10m, 1km or 100km remote from the at least one Bragg grating.

In one specific embodiment of the present invention the apparatus comprises at least two optical light guides, each optical light guide incorporating a series of Bragg gratings. At least one first optical light guide is positioned so that the incorporated Bragg gratings are coupled to respective movable wall portions and at least one second optical light guide may be arranged so that the incorporated Bragg gratings are positioned in the proximity of the Bragg gratings of the first optical light guide and largely only experience a change in strain if the local temperature changes. In this case the apparatus typically is arranged so that reflections form the Bragg gratings of the at least one second optical light guide are used for correcting temperature related effects associated with reflections from the Bragg gratings of the at least one first optical light guide.

In an embodiment an apparatus for pressure sensing, the apparatus comprising:
a light guide incorporating at least one Bragg grating;
at least one moveable wall portion having opposite first and second sides, the at least one moveable wall portion being positioned so that a change in pressure at one of the sides relative to a pressure at the other side will move the moveable wall portion, the at least one moveable wall portions being coupled to the or a respective Bragg grating so that the movement of one of the moveable wall portions causes a force on the respective Bragg grating resulting in a change in strain of the respective Bragg grating; and
a tube having an internal space in which at least a portion of the light guide with the at least one Bragg grating is positioned or which is in fluidal communication with an internal space at the at least one optical fibre, the tube having an open end-portion;
wherein the internal space at the at least one Bragg grating is in fluidal communication with the open end portion of the tube.

The apparatus typically is arranged for in-vivo pressure measurements and the tube typically has an open end that is arranged to be located outside a body during the in-vivo pressure measurements so that the at least one internal space at the at least one Bragg grating is in fluidal communication with a space outside the body. The other end of the tube typically is closed.

The tube typically is a flexible tube.

The at least one moveable wall portion may be a portion of the tube or may be provided in addition to the tube.

The invention will be more fully understood from the following description of specific embodiments of the invention. The description is provided with reference to the accompanying drawings.

### Brief Description of the Drawings

Figure 1 (a) and (b) shows a system for distributed pressure sensing according to a specific embodiment of the present invention,
Figures 2 (a) and (b) show components of an apparatus for distributed pressure sensing according to an embodiment of the present invention,
Figure 3 shows components of an apparatus for pressure sensing according to another specific embodiment of the present invention and
Figure 4 shows components of an apparatus for pressure sensing according to further specific embodiment of the present invention.

### Detailed Description of Specific Embodiments

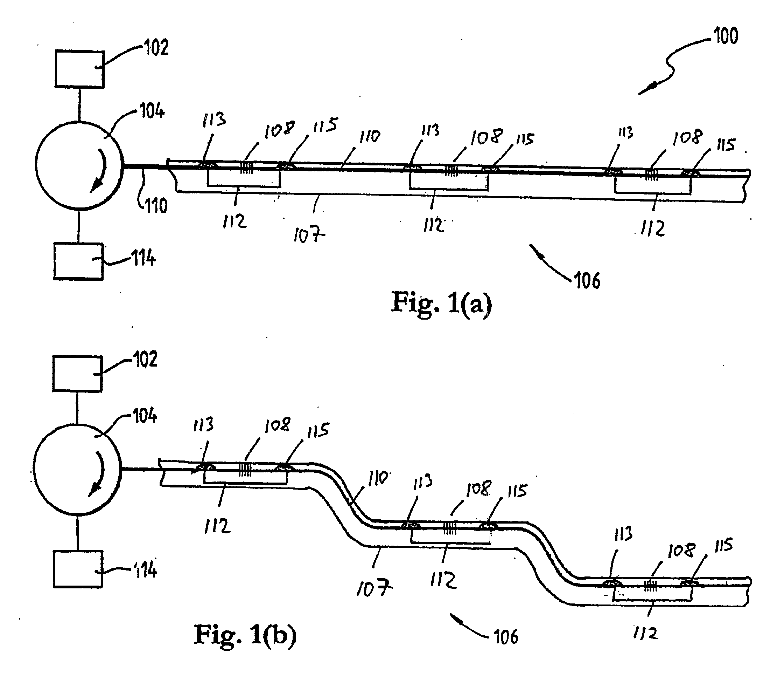

Referring initially to Figure 1 (a), a system for distributed pressure sensing according to a specific embodiment of the present invention is now described. The system 100 comprises a light source 102 which in this embodiment is a broadband light source commonly referred to as a "white" light source even though the light that is emitted by the light source 102 may have any wavelength range.

The light is directed via optical circulator 104 to an apparatus for distributed pressure sensing 106. In a variation of this embodiment the circulator 104 may be replaced by an optical coupler, an optical splitter or an optical beam splitter.

The apparatus 106 comprises a catheter 107 which is formed from a bio-compatible flexible material and arranged for insertion into a lumen of the human body. Further, the apparatus 106 typically comprises an X-ray opaque material, such as a metallic material, for locating the apparatus 106 in the human body.

The apparatus 106 comprises a series of Bragg gratings 108 which are formed in an optical fibre 110. Each Bragg grating 108 is in this embodiment positioned in association with rigid members 112. In this embodiment, the optical fibre 110 is rigidly connected to end-portions 113 and 115 of a respective rigid member 112 so that respective Bragg gratings 108 are positioned between the end-portions 113 and 115 and.respective sensing region are defined. Each Bragg grating 108 is positioned in the proximity of the catheter 107 such that a local external pressure change effects a strain or a change in strain of a respective Bragg grating 108. The strain on each Bragg grating causes a change of an optical property of the Bragg gratings 108, such as a change of an optical path length, which influences an optical response of the gratings 108 to light reflected from the Bragg grating 108.

In a variation of the embodiment described above and illustrated in Figure 1, the apparatus may also comprise individual flexible diaphragms, such as silicone diaphragms, which are positioned between the Bragg gratings 108 and portions of the catheter 107. Each Bragg grating 108 may alternatively also be positioned within or over the individual diaphragms.

In further variation, the apparatus may comprise an additional tube in which the light guide 110 with the Bragg gratings 108 and the rigid members 112 are positioned. The tube is formed from a flexible material, such as silicone or a related polymeric material and is positioned in the catheter 107. It will be appreciated, that alternatively each Bragg grating 108 may be positioned within or over the additional tube that is positioned within the catheter 107.

As the optical fibre 110 is attached to the end-portions 113 and 115 of each rigid member 112, the rigid members 112 prevent that a strain of one of the sensing regions is affected by a change in axial, transverse, or otherwise directed strain at a fibre portion outside that sensing region (for example at another sensing region). Consequently, it is possible to conduct distributed pressure measurements at a plurality of locations and independent from one another, even in an environment such as the oesophagus in which an axial strain is inherently applied to the sensor array.

In this embodiment the apparatus 106 comprises a series of three Bragg gratings 108. In alternative embodiments the apparatus 106 may comprise two or more than three Bragg gratings at any fixed or variable pitch.

In the embodiment described above and illustrated in Figure 1 each Bragg grating 108 of the series has a slightly different refractive index variation so that each Bragg grating 108 has an optical response that has a slightly different spectral distribution. The light that is produced by light source 102 and that is directed to the Bragg gratings 108 therefore causes three unique responses from the Bragg gratings 108 which are directed via the optical circulator 104 to optical analyser 114 for optical analysis. Such a procedure is commonly referred to as wavelength division multiplexing (WDM). The Bragg gratings may also effect optical responses which overlap in wavelength or frequency space as long as sufficient information is known about each Bragg grating to allow the signals to be successfully deconvolved.

As in this embodiment each Bragg grating 108 causes a different response, it is possible to associate a particular response with a position along the apparatus 106 to perform distributed pressure measurements and detect relative pressure differences between the positions of the Bragg gratings 108 in the series. The combined response from the Bragg gratings is wavelength division multiplexed and the optical analyser 114 uses known wavelength division de-multiplexing techniques to identify the responses from the respective grating positions. Suitable software routines are used to determine a pressure or pressure distribution from the optical responses received from the Bragg gratings. Pressure measurements typically include calibrating the apparatus.

In a variation of this embodiment at least some of the Bragg gratings 108 may be identical and consequently, if the strain conditions are the same, their optical response will also be the same. In this case a pulsed light source may be used to guide light to the Bragg gratings and the positions of the Bragg gratings may be estimated from a time at which the responses are received by the optical analyser 114.

In one particular example the reflectivity of each Bragg grating 108 is chosen so that each response has, at the location of the optical analyser 114, approximately the same intensity.

It will be appreciated that in a further variation of this embodiment the apparatus may be arranged so that responses from respective Bragg gratings can be analysed by receiving light that is transmitted through the Bragg gratings 108. For example, in this case the apparatus 106 typically is arranged so that light is guided from the light source 102 through the Bragg gratings 108 and then directly to the optical analyser 114. In such an embodiment the fibre may be looped back on itself so that both light source and detector can be located at a common end of the device

Each Bragg grating 108 is written into the optical fibre which comprises spliced fibre portions. It will be appreciated, that in alternative embodiments the Bragg gratings 108 and the fibre portions 110 may be integrally formed from one optical fibre. The same optical fibre may be used for writing respective refractive index variations for each grating so that spaced apart Bragg gratings are formed separated by fibre portions.

Portions of the optical fibre that are located between the rigid members 112 are relatively flexible and consequently the apparatus 106 is an articulated device. Figure 1 (b) shows the system for pressure sensing 100 also shown in Figure 1 (a), but, between adjacent sensing regions the optical fibre is bent.

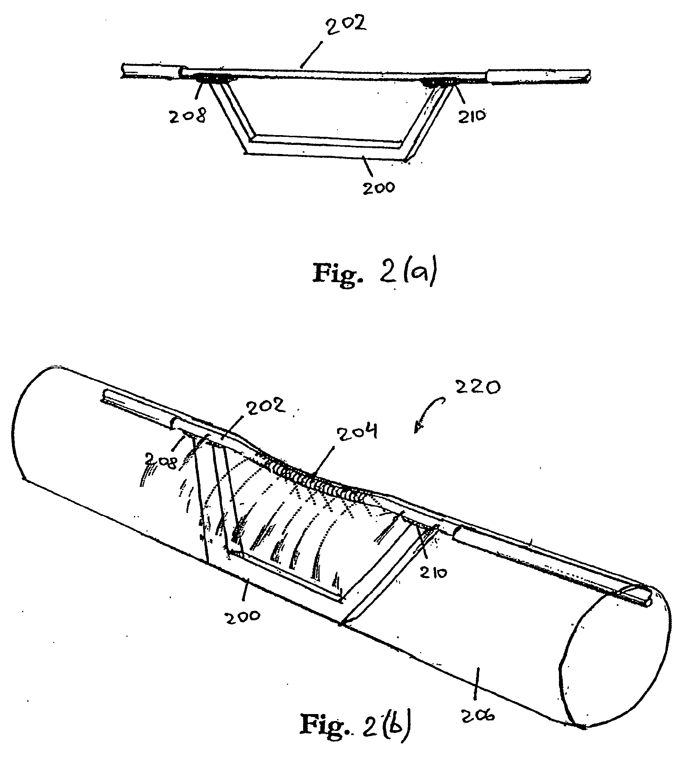

Figures 2 (a) and (b) show schematically components of an apparatus for pressure sensing in more detail. The apparatus for distributed pressure sensing comprises a series of rigid members 200 and, written in an optical fibre 202, a series of Bragg gratings 204. The rigid members 200 and the optical fibre 202 are positioned in a flexible tube 206 which may be a catheter or which itself may be positioned in a catheter.

The Bragg gratings 204 are formed in the optical fibre 202 that comprises a core/cladding region and a protective coating.

The optical fibre 202 is attached to the rigid members 200 at attachment regions 208 and 210 so that each Bragg grating 204 is positioned between respective attachment regions 208 and 210 and so that the waveguide containing the grating is rigidly fixed in space relative to the rigid member). In this embodiment attachment is effected using a suitable glue, but a person skilled in the art will appreciate that various other means may be used to secure the optical fibre 202 to the rigid members 200.

The flexible tube may have at least one open end. A change in external pressure in the proximity of one of the Bragg gratings (and away from the open end or ends, if present, of the tubular portion 206) will result in a deflection of the tubular portion at the respective Bragg grating 204. This results in a force on the respective Bragg grating 204 between the attachment regions 208 and 210. In this embodiment the Bragg grating 204 is distorted into the internal space of the flexible tube 206 and consequently an increase in external pressure will increase that distortion.

In one embodiment the flexible tube 206 comprises a cold or hot shrink tube which is located over the Bragg gratins 204 and which is positioned in an external catheter. In alternative embodiments the flexible tube 206 is formed from any other suitable flexible polymeric material such as silicone. The rigid members 200 may be formed from silicon, a plastics or metallic material, or any other suitable rigid material.

An optical response of the Bragg grating typically has a linear dependency on the temperature and on axial strain, but the strain on the optical fibre 202 between the attachment regions 208 and 210 and enclosed in a diaphragm of suitable strain and thermal properties typically has a nonlinear dependency on the temperature. Consequently, if a Bragg grating is arranged so that a change in temperature of the enclosure also causes a change in strain, the optical response of the Bragg grating will have a combined non-linear and linear dependency on the temperature.

In this example the distortion of the Bragg gratings 204 and the design of the rigid members 200 are selected so that the optical response of the Bragg grating does not change by more than approximately 0.001nm if the temperature changes by ± 1 degree from the normal operating temperature of the apparatus centred at approximately 37°C.

In the example of the units 220 shown in Figure 2(b) the distortion of the Bragg grating 202 causes a tensile strain of the Bragg grating 204. If the ambient temperature now increases from the normal operation temperature, a number of physical effects may take place. The optical period of the Bragg grating 204 will typically increase. Further, the flexible tube 206, which is arranged to distort the optical fibre 202 in the proximity of the Bragg gratings 204 (at a normal operating temperature), will tend to expand and/or the Young's modulus of the material of the flexible tube 206 will change which in turn causes a change of the distorting force on the Bragg grating 204. Hence, it is possible to influence the temperature dependency of optical responses by selecting materials having selected thermal behaviour.

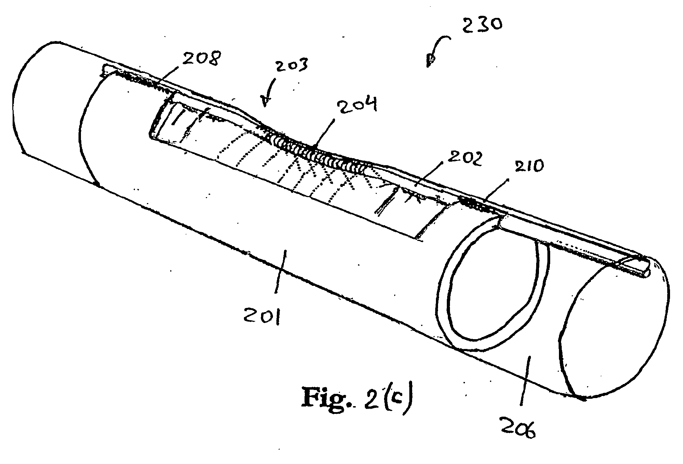

Figure 2 (c) shows a unit for pressure sensing which is related to the unit 220 shown in Figure 2(b). The unit 230, however, comprises a cylindrical rigid member 201 which is attached the optical fibre 210 at attachment regions 208 and 210. The rigid member 201 has a window 203 into which the Bragg grating 204 is distorted by the tube 206.

The units shown in Figure 2(a), (b) and (c) may form a part of an apparatus for distributed pressure sensing. However, a person skilled in the art will appreciate that alternatively the apparatus for pressure sensing may only comprise one such unit which enables pressure sensing at one location.

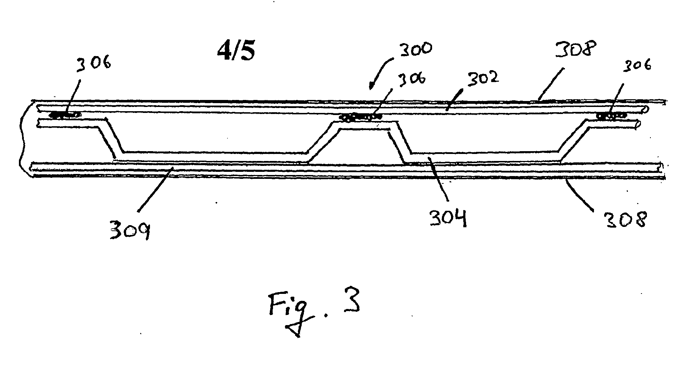

Figure 3 shows components of an apparatus for pressure sensing according to another embodiment of the present invention. The apparatus 300 comprises an optical fibre 302 that is attached to a rigid member 304 at attachment regions 306. The optical fibre comprises Bragg gratings which are positioned between adjacent attachment regions 306. The rigid member 304 with the optical fibre 302 is positioned in a flexible tube 308 which may be, or may be positioned in, a flexible external catheter. In this embodiment, the apparatus 300 comprises only one rigid member 304 which may for example be provided in form of a suitably bent rod such as a metallic rod. In this example the apparatus 300 is not an articulated device, but has the advantage of particularly simple manufacture.

The apparatus 300 comprises in this embodiment also a second optical fibre 309, that also includes Bragg gratings and typically has properties that are very similar to those of the optical fibre 308. The optical fibre 309 comprises Bragg gratings that are positioned in the proximity of the Bragg gratings of the optical fibre 308. However, the optical fibre 309 is not exposed to a change in force if an external pressure changes. For example, the optical fibre 309 may be aligned with a straight portion of the rigid member 304 or another means may be used to prevent that an external pressure change affects the optical fibre 309. The optical fibre typically is also attached at attachment regions (not shown) to the rigid member so that a local change in strain at one of the Bragg gratings does not effect a local change in strain of an adjacent Bragg grating. Consequently, each Bragg grating of the optical fibre 309 will only experience a change in strain caused by a local change in temperature and optical signals reflected by the Bragg gratings of the optical fibre 309 can be used to correct the signals received from the Bragg grating 308 for temperature related effects.

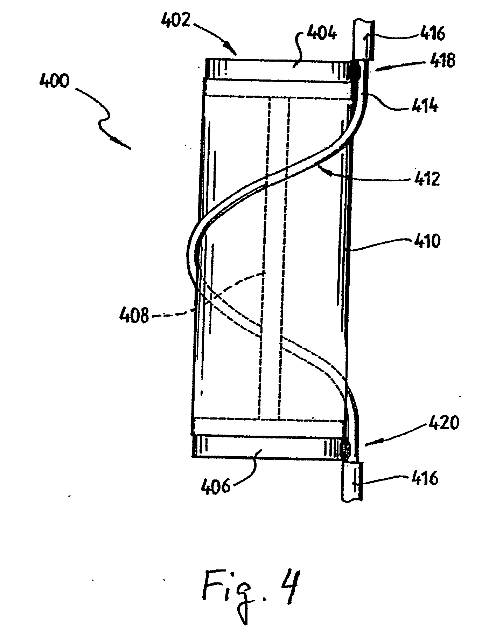

The examples of the components of the apparatus for pressure sensing shown in Figures 2 and 3 are suitable for asymmetric pressure sensing. Figure 4 shows a component of an apparatus for pressure sensing according to a further embodiment of the present invention which can be used for more symmetric pressure measurements. The apparatus for pressure sensing comprises a series of the components 400 which are optically coupled for example by an optical fibre.

The component 400 shown in Figure 4 comprises a rigid structure 402 having rigid upper and lower portions 404 and 406 and a rigid support portion 408 connecting the upper and lower portions 404 and 406. The rigid support portion is surrounded by a diaphragm 410 which is applied to the upper and lower portions 404 and 406 so that an enclosed internal volume is formed. The component 400 also comprises a Bragg grating 412 and a core/cladding region 414. The core/cladding region 414 is attached to the upper and lower portions 404 and 406 at positions 418 and 420. In this embodiment the core/cladding region is glued at these positions to the upper and lower portions 404 and 406 respectively, and attached to the diaphragm 410. The rigid support structure has perforations or holes in the cylindrical upper and lower portions 404 and 406. A series of the components 400 typically are positioned in an external catheter suitable for insertion into a body lumen. The apparatus for pressure sensing comprises in this embodiment a series of the components 400 which are positioned in an external catheter (not shown).

For example, the Bragg grating 412 may be attached to the diaphragm 410 using a flexible adhesive. If a pressure in a region adjacent the diaphragm 410 changes, the diaphragm 410 will move which will cause a strain in the Bragg grating 412 and therefore the pressure change can be sensed. As the Bragg grating 412 is wound around the diaphragm 410 and the diaphragm 410 surrounds the support 408 so that internal volume is formed between the support 408 and the diaphragm 410, a pressure change can be sensed at any position around the diaphragm 410 using the device 400. Similar to the embodiments discussed before, the Bragg grating 412 is slightly distorted into the enclosed volume (the distortion into the enclosed volume at the normal operating temperature is not shown in Figure 4).

The rigid portions 402, 404 and the support 408 typically are composed of silicon or of any other suitable rigid material including plastics or metallic materials. The diaphragm 410 typically is a thin layer having a thickness of the order of 0.1mm being composed of silicone, another polymeric material or a metallic material.

Although the invention has been described with reference to particular examples, it will be appreciated by those skilled in the art that the invention may be embodied in many other forms. For example, the apparatus for pressure sensing may comprise Bragg gratings that are positioned within or on diaphragms. Further, it is to be appreciated that alternatively the rigid members may be positioned between adjacent sensing regions.

## Claims

1. An apparatus (106) for distributed pressure sensing, the apparatus (106) comprising:
at least one first optical light guide (302) and at least one second optical light guide (309), each optical light guide incorporating a series of Bragg gratings (108; 204);
a plurality of moveable wall portions having opposite first and second sides, each moveable wall portion being positioned so that a change in pressure at one of the sides relative to a pressure at the other side will move the moveable wall portion, the moveable wall portions being coupled to respective Bragg gratings (108; 204) of the at least one first optical light guide (302) so that the movement of one of the moveable wall portions causes a force on the respective Bragg grating (108; 204) resulting in a change in strain of the respective Bragg grating;
a flexible tube (206) having an internal space in which portions of the light guides (302, 309) including the Bragg gratings (108; 204) are positioned, the flexible tube having an open end-portion, a portion of the internal space at each Bragg grating (108; 204) being in fluidal communication with the open end-portion such that a local increase in internal pressure in the internal space caused by a suitable movement of the movable wall portion is reduced;
at least one rigid member (112; 200) that is attached to the at least one first optical light guide (302) at attachment regions (113, 115; 208, 210) between which sensing regions of the Bragg gratings (108; 204) are defined so that a series of the sensing regions is formed, the at least one rigid member (112; 200) being attached to the at least one first optical light guide (302) at attachment regions (113, 115; 208, 210) at either end of each sensing region,
wherein each moveable wall portion is either a portion of the flexible tube or is provided in addition to the flexible tube; **characterised in that**
the apparatus (106) is arranged for in-vivo pressure measurements and the open end-portion of the tube (206) is arranged to be located outside a body during the in-vivo pressure measurements so that the internal spaces of the tube at the Bragg gratings (108; 204) are in fluidal communication with a space outside the body and
the at least one second optical light guide (309) is arranged so that the incorporated Bragg gratings (108; 204) are positioned in the proximity of the Bragg gratings (108; 204) of the first optical light guide (302) and only experience a change in strain if the local temperature changes.

2. The apparatus (106) of claim 1 wherein the Bragg gratings (108; 204) of the at least one first optical light guide (302) are distorted into the internal space of the tube (206).

3. The apparatus (106) of any one of the preceding claims wherein the at least one rigid member (112; 200) is arranged so that a strain in the sensing regions is not influenced by a change in strain of the light guide outside the sensing regions.

4. The apparatus (106) of any one of the preceding claims wherein the moveable wall portions are wall portions of the tube (206).

5. The apparatus (106) of claim 4 wherein the tube is provided in form of a catheter (107).

6. The apparatus (106) as claimed in any one of claim 3 wherein the apparatus (106) comprises a catheter (107) and wherein each moveable wall portion is an individual diaphragm and the individual diaphragms with the at least one rigid member (112; 200) together with at least a portion of the light guides (302, 304) are positioned in a catheter (107).

7. The apparatus (106) as claimed in any one of the preceding claims comprising an X-ray opaque material, which enables imaging of the position of the apparatus (106) in the human body.

8. The apparatus (106) as claimed in any one of the preceding claims wherein the force on a respective Bragg grating (108; 204) is a force on a side portion of the respective Bragg grating (108; 204).

9. The apparatus (106; 300) as claimed in any one of the preceding claims arranged so that reflections form the Bragg gratings (108; 204) of the at least one second optical light guide (309) are used for correcting temperature related effects associated with reflections from the Bragg gratings (108; 204) of the at least one first optical light guide (302).

10. The apparatus (106) of any one of claims 1 to 3 wherein the at least one moveable wall portion is provided in addition to the tube (206).

## Patentansprüche

1. Vorrichtung (106) zum Erfassen von verteiltem Druck, wobei die Vorrichtung (106) Folgendes umfasst:
wenigstens einen ersten optischen Lichtleiter (302) und wenigstens einen zweiten optischen Lichtleiter (309), wobei jeder optische Lichtleiter eine Serie von Bragg-Gittern (108; 204) beinhaltet;
mehrere bewegliche Wandabschnitte mit gegenüberliegenden ersten und zweiten Seiten, wobei jeder bewegliche Wandabschnitt so positioniert ist, dass eine Druckänderung auf einer der Seiten relativ zu einem Druck auf der anderen Seite den beweglichen Wandabschnitt bewegt, wobei die beweglichen Wandabschnitte mit jeweiligen Bragg-Gittern (108; 204) des wenigstens einen ersten optischen Lichtleiters (302) gekoppelt sind, so dass eine Bewegung eines der beweglichen Wandabschnitte bewirkt, dass eine Kraft auf dem jeweiligen Bragg-Gitter (108; 204) zu einer Änderung der Spannung des jeweiligen Bragg-Gitters führt;
eine flexible Röhre (206) mit einem Innenraum, in dem Abschnitte der Lichtleiter (302, 309) einschließlich der Bragg-Gitter (108; 204) positioniert sind, wobei die flexible Röhre einen offenen Endabschnitt aufweist, wobei ein Abschnitt des Innenraums an jedem Bragg-Gitter (108; 204) in Fluidverbindung mit dem offenen Endabschnitt ist, so dass eine durch eine geeignete Bewegung des beweglichen Wandabschnitts bewirkte lokale Zunahme des Innendrucks im Innenraum reduziert wird;
wenigstens ein starres Element (112; 200), das an dem wenigstens einen ersten optischen Lichtleiter (302) in Anbringungsregionen (113, 115; 208, 210) angebracht ist, zwischen denen Erfassungsregionen der Bragg-Gitter (108; 204) definiert sind, so dass eine Serie der Erfassungsregionen entsteht, wobei das wenigstens eine starre Element (112; 200) an dem wenigstens einen ersten optischen Lichtleiter (302) an Anbringungsregionen (113, 115; 208, 210) an beiden Enden jeder Erfassungsregion angebracht ist,
wobei jeder bewegliche Wandabschnitt entweder ein Abschnitt der flexiblen Röhre ist oder zusätzlich zu der flexiblen Röhre vorgesehen ist; **dadurch gekennzeichnet, dass**
die Vorrichtung (106) für In-vivo-Druckmessungen ausgelegt ist und der offene Endabschnitt der Röhre (206) so ausgelegt ist, dass er sich während der In-vivo-Druckmessungen außerhalb eines Körpers befindet, so dass die Innenräume der Röhre an den Bragg-Gittern (108; 204) in Fluidverbindung mit einem Raum außerhalb des Körpers ist, und
der wenigstens eine zweite optische Lichtleiter (309) so ausgelegt ist, dass die integrierten Bragg-Gitter (108; 204) in der Nähe der Bragg-Gitter (108; 204) des ersten optischen Lichtleiters (302) positioniert sind und lediglich dann eine Spannungsänderung erfahren, wenn sich die lokale Temperatur ändert.

2. Vorrichtung (106) nach Anspruch 1, wobei die Bragg-Gitter (108; 204) des wenigstens einen ersten optischen Lichtleiters (302) in den Innenraum der Röhre (206) verzerrt sind.

3. Vorrichtung (106) nach einem der vorherigen Ansprüche, wobei das wenigstens eine starre Element (112; 200) so angeordnet ist, dass eine Spannung in den Erfassungsregionen durch eine Spannungsänderung des Lichtleiters außerhalb der Erfassungsregionen nicht beeinflusst wird.

4. Vorrichtung (106) nach einem der vorherigen Ansprüche, wobei die beweglichen Wandabschnitte Wandabschnitte der Röhre (206) sind.

5. Vorrichtung (106) nach Anspruch 4, wobei die Röhre in Form eines Katheters (107) bereitgestellt wird.

6. Vorrichtung (106) nach einem der Ansprüche 3, wobei die Vorrichtung (106) einen Katheter (107) umfasst und wobei jeder bewegliche Wandabschnitt eine individuelle Membran ist und die individuellen Membranen mit dem wenigstens einen starren Element (112; 200) zusammen mit wenigstens einem Abschnitt der Lichtleiter (302, 304) in einem Katheter (107) positioniert sind.

7. Vorrichtung (106) nach einem der vorherigen Ansprüche, die ein für Röntgenstrahlen opakes Material umfasst, das eine Abbildung der Position der Vorrichtung (106) im menschlichen Körper ermöglicht.

8. Vorrichtung (106) nach einem der vorherigen Ansprüche, wobei die Kraft auf einem jeweiligen Bragg-Gitter (108; 204) eine Kraft auf einem Seitenabschnitt des jeweiligen Bragg-Gitters (108; 204) ist.

9. Vorrichtung (106; 300) nach einem der vorherigen Ansprüche, die so ausgelegt ist, dass Reflexionen die Bragg-Gitter (108; 204) des wenigstens einen zweiten optischen Lichtleiters (309) bilden zum Korrigieren von temperaturbezogenen Effekten in Verbindung mit Reflexionen von den Bragg-Gittern (108; 204) des wenigstens einen ersten optischen Lichtleiters (302) benutzt werden.

10. Vorrichtung (106) nach einem der Ansprüche 1 bis 3, wobei der wenigstens eine bewegliche Wandabschnitt zusätzlich zu der Röhre (206) bereitgestellt wird.

## Revendications

1. Appareil (106) pour la détection de la pression distribuée, l'appareil (106) comprenant :
au moins un premier guide de lumière optique (302) et au moins un deuxième guide de lumière optique (309), chaque guide de lumière optique incorporant une série de réseaux de Bragg (108 ; 204) ;
plusieurs parties de paroi mobiles, comportant des premiers et deuxièmes côtés opposés, chaque partie de paroi mobile étant positionnée de sorte qu'un changement de la pression au niveau de l'un des côtés par rapport à une pression au niveau de l'autre côté entraîne le déplacement de la partie de paroi mobile, les parties de paroi mobiles étant accouplées à des réseaux de Bragg respectifs (108 ; 204) du au moins un guide de lumière optique (302), de sorte que le déplacement de l'une des parties de paroi mobiles entraîne l'application d'une force au réseau de Bragg respectif (108 ; 204), résultant en un changement de la contrainte du réseau de Bragg respectif ;
un tube flexible (206), comportant un espace interne dans lequel sont positionnées des parties des guides de lumière (302 ; 309) englobant les réseaux de Bragg (108 ; 204), le tube flexible comportant une partie d'extrémité ouverte, une partie de l'espace interne au niveau de chaque réseau de Bragg (108 ; 204) étant en communication de fluide avec la partie d'extrémité ouverte, de sorte qu'un accroissement local de la pression interne dans l'espace interne, entraîné par un déplacement approprié de la partie de paroi mobile, est réduit ;
au moins un élément rigide (112 ; 200) fixé sur le au moins un guide de lumière optique (302) au niveau de régions de fixation (113, 115 ; 208, 210) entre lesquelles sont définies des régions de détection des réseaux de Bragg (108 ; 204), de sorte à former une série de régions de détection, le au moins un élément rigide (112 ; 200) étant fixé sur le au moins un premier guide de lumière optique (302) au niveau de régions de fixation (113, 115 ; 208, 210) au niveau de chaque extrémité de chaque région de détection ;
dans lequel chaque partie de paroi mobile est une partie du tube flexible ou est prévue en plus du tube flexible ; **caractérisé en ce que**
l'appareil (106) est destiné à des mesures de la pression in vivo et la partie d'extrémité ouverte du tube (206) est destinée à être positionnée à l'extérieur d'un corps au cours des mesures de la pression in vivo, de sorte que les espaces internes du tube au niveau des réseaux de Bragg (108 ; 204) sont en communication de fluide avec un espace à l'extérieur du corps ; et
le au moins un deuxième guide de lumière optique (309) est agencé de sorte que les réseaux de Bragg incorporés (108 ; 204) sont positionnés à proximité des réseaux de Bragg (108 ; 204) du premier guide de lumière optique (302) et subissent uniquement un changement de la contrainte lors d'un changement de la température ambiante.

2. Appareil (106) selon la revendication 1, dans lequel les réseaux de Bragg (108 ; 204) du au moins un premier guide de lumière optique (302) sont déformés dans l'espace interne du tube (206).

3. Appareil (106) selon l'une quelconque des revendications précédentes, dans lequel le au moins un élément rigide (112 ; 200) est agencé de sorte qu'une contrainte dans les régions de détection n'est pas influencée par un changement de la contrainte du guide de lumière à l'extérieur des régions de détection.

4. Appareil (106) selon l'une quelconque des revendications précédentes, dans lequel les parties de paroi mobiles sont des parties de paroi du tube (206).

5. Appareil (106) selon la revendication 4, dans lequel le tube a la forme d'un cathéter (107).

6. Appareil (106) selon l'une quelconque de la revendication 3, dans lequel l'appareil (106) comprend un cathéter (107) et dans lequel chaque partie de paroi mobile est une membrane individuelle, les membranes individuelles avec le au moins un élément rigide (112 ; 200), ensemble avec au moins une partie du guide de lumière (302, 304), étant positionnés dans un cathéter (107).

7. Appareil (106) selon l'une quelconque des revendications précédentes, comprenant un matériau opaque aux rayons X, permettant la formation d'une image de la position de l'appareil (106) dans le corps humain.

8. Appareil (106) selon l'une quelconque des revendications précédentes, dans lequel la force exercée sur un réseau de Bragg respectif (108 ; 204) est une force appliquée à une partie latérale du réseau de Bragg respectif (108 ; 204).

9. Appareil (106 ; 300) selon l'une quelconque des revendications précédentes, agencé de sorte que les réflexions forment les réseaux de Bragg (108 ; 204) du au moins un deuxième guide de lumière optique (309) sont utilisées pour corriger les effets relatifs à la température associés aux réflexions provenant des réseaux de Bragg (108 ; 204) du au moins un premier guide de lumière optique (302).

10. Appareil (106) selon l'une quelconque des revendications 1 à 3, dans lequel la au moins une partie de paroi mobile est prévue en plus du tube (206).
